# EUROPEAN PATENT SPECIFICATION

(11) **EP 4 218 745 B1**
(45) Date of publication and mention of the grant of the patent: **05.03.2025**
(21) Application number: 22188635.1
(22) Date of filing: 03.08.2022
(51) Int. Cl.: A61K 31/121, C12N 15/86, C12N 15/90, A61K 38/17, A61P 5/00, A61P 9/00, A61P 25/00, C07K 14/705, C12N 15/85

(54) **USE OF 2-PENTANONE AND SPECIFIC RECEPTOR THEREOF IN MANUFACTURE OF PRODUCTS REGULATING CELL FUNCTIONS**
VERWENDUNG VON 2-PENTANON UND DESSEN SPEZIFISCHEM REZEPTOR ZUR HERSTELLUNG VON PRODUKTEN ZUR REGULIERUNG VON ZELLFUNKTIONEN
UTILISATION DE 2-PENTANONE ET DE SON RÉCEPTEUR SPÉCIFIQUE DANS LA FABRICATION DE PRODUITS RÉGULANT LES FONCTIONS CELLULAIRES

(30) Priority: 27.01.2022 CN 202210102995
(43) Date of publication of application: 02.08.2023
(73) Proprietor: Taihe Hospital of Shiyan City (Affiliated Hospital of Hubei University of Medecine), Shiyan 442001 (CN)
(72) Inventor: KE, Changbin, Shiyan, 442001 (CN); WU, Yanqiong, Shiyan, 442001 (CN); SU, Shanchun, Shiyan, 442001 (CN); XU, Xueqin, Shiyan, 442001 (CN)
(74) Representative: Bayramoglu et al.

(56) References cited:
- US-A1- 2005 008 714
- US-A1- 2020 240 978
- US-B2- 8 822 165
- DANIEL J. URBAN ET AL: "DREADDs (Designer Receptors Exclusively Activated by Designer Drugs): Chemogenetic Tools with Therapeutic Utility", ANNUAL REVIEW OF PHARMACOLOGY AND TOXICOLOGY., vol. 55, no. 1, 6 January 2015 (2015-01-06), US, pages 399 - 417, XP055619255, ISSN: 0362-1642, DOI: 10.1146/annurev-pharmtox-010814-124803
- RICHARD BENTON ET AL: "Variant Ionotropic Glutamate Receptors as Chemosensory Receptors in Drosophila", CELL, vol. 136, no. 1, 1 January 2009 (2009-01-01), Amsterdam NL, pages 149 - 162, XP055287086, ISSN: 0092-8674, DOI: 10.1016/j.cell.2008.12.001
- HOU CHANGJUN ET AL: "Development of Cell-Based Olfactory Biosensors", ANALYTICAL LETTERS, vol. 45, no. 2-3, 1 January 2012 (2012-01-01), US, pages 202 - 218, XP093008604, ISSN: 0003-2719, DOI: 10.1080/00032719.2011.633191
- LORE M PEETERS ET AL: "Combining designer receptors exclusively activated by designer drugs and neuroimaging in experimental models: A powerful approach towards neurotheranostic applications", BRITISH JOURNAL OF PHARMACOLOGY, WILEY-BLACKWELL, UK, vol. 177, no. 5, 3 February 2020 (2020-02-03), pages 992 - 1002, XP071056883, ISSN: 0007-1188, DOI: 10.1111/BPH.14885
- ZHANG JI ET AL: "Utilizing Designed Receptors Exclusively Activated by Designer Drug Chemogenetic Tools to Identify Beneficial G Protein-Coupled Receptor Signaling for Fibrosis", JOURNAL OF PHARMACOLOGY AND EXPERIMENTAL THERAPEUTICS, vol. 375, no. 2, 1 November 2020 (2020-11-01), US, pages 357 - 366, XP093008677, ISSN: 0022-3565, Retrieved from the Internet <URL:https://jpet.aspetjournals.org/content/jpet/375/2/357.full.pdf> DOI: 10.1124/jpet.120.000103
- BECNEL JAIME ET AL: "DREADDs in Drosophila: A Pharmacogenetic Approach for Controlling Behavior, Neuronal Signaling, and Physiology in the Fly", CELL REPORTS, vol. 4, no. 5, 1 September 2013 (2013-09-01), US, pages 1049 - 1059, XP093009070, ISSN: 2211-1247, DOI: 10.1016/j.celrep.2013.08.003
- SU SHANCHUN ET AL: "A non-invasive, fast on/off "Odourgenetic" Method to Manipulate Physiology", BIORXIV, 25 November 2022 (2022-11-25), XP093008393, Retrieved from the Internet <URL:https://www.biorxiv.org/content/10.1101/2022.11.25.517891v1.full.pdf> [retrieved on 20221215], DOI: 10.1101/2022.11.25.517891

## Description

### TECHNICAL FIELD

The present disclosure relates to the field of biotechnology, and specifically to use of 2-pentanone and specific receptor thereof in the manufacture of products regulating cell functions.

### BACKGROUND

In the field of fundamental life science study, neurons, cardiomyocytes and skeletal muscle cells are able to release or transmit action potentials, and thus have important physiological functions in life processes. For in-depth studies, researchers usually activate neurons in a particular area of the brain, or simulate cardiomyocytes, skeletal muscle cells and intracellular calcium-dependent physiological functions (e.g., cell secretion), by some means, to activate certain particular behavior or to produce some important physiological functions. Application of such techniques plays an important role in the treatment of diseases including mental diseases, neurological disorders, myocardial dysfunction, motor dysfunction and endocrine disorders.

Optogenetics and chemogenetics have been commonly used in these field. Optogenetics requires cells in a target area to express light-sensitive channel proteins such as ChR2 and NpHR, and an optical fiber connected to an external laser is embedded in this area, so that the laser beam with a specific wavelength activates the corresponding light-sensitive channel proteins in that area, so as to excite or inhibit the cells in that area. Optogenetics is advantaged in that it precisely controls the treatment time or intensity and exhibits high efficiency and stable effects, owing to using laser. The treatment based on optogenetics, however, cannot be carried out for a long time, due to heat generation from laser beams. In addition, the optogenetics requires accessories such as optical fibers, laser controllers and catheters in its use, and to embed optical fibers is delicate and complicated, which cause difficulty in operation and many complications. Moreover, heads of the optical fibers need to be fixed inside the cranium of animals during the later experiments, which brings inconvenience to them and also injury to cells by laser itself. Therefore, it is inconvenient to clinically apply optogenetics.

Chemogenetics requires cells in a target area to express artificially engineered chemoreceptors such as hM3Dq and hM4Di, in which the receptors expressed by the cells in the target area are activated by feeding or injecting agonists (Clozapine-N-Oxide, CNO) of these receptors into the body, to excite or inhibit activity of the cells. Chemogenetics needs less instruments, can be simply operated in experiments and processed for a long time, and thus has a promising clinical application. However, chemogenetics takes effect very slowly (more than 0.5 hours), failing to control time and intensity accurately, and failing to work rapidly and to be eluted out rapidly, due to metabolic properties of CNO in the body.

In summary, the conventional optogenitics and chemogenetics are disadvantaged in controlling the activity of cells, since they are not suitable for long-term use, complicated in operations, inconvenient, and lacking of accuracy, and also cause difficult for cells to recover. Patent document US 8 822 165 B2 discloses the use of 2-pentanone in combination with the mouse olfactory receptor Olfr168 or its chimeras for producing cAMP in a cell. Patent document US 2020/240978 A1 discloses that one of the odor molecules which can activate Olfr168 or Olfr205 is 2-pentanone. Non patent literature XP55619255A (DANIEL J. URBAN ET AL., ANNUAL REVIEW OF PHARMACOLOGY AND TOXICOLOGY, 2015) discloses DREADDs (designer receptors exclusively activated by designer drugs) have been used ubiquitously to modulate GPCR activity non-invasively in vivo. Non patent literature XP55287086A (RICHARD BENTON ET AL., CELL, 2009) discloses Ors and Irs are expressed in developmentally distinct sensory lineages in the antenna. Patent documnet US 20050008714 A1 discloses compositions containing one or more plant essential oils for controlling insects and methods for using these compositions.

### SUMMARY

In view of the forgoing, embodiments of the present disclosure provide a 2-pentanone receptor and use thereof, so as to solve the problems, of the conventional optogenitics and chemogenetics for controlling the physiological activities of cells, that they are not suitable for long-term use, complicated in operations, inconvenient, and lacking of accuracy.

In order to achieve the above object, the present invention provides the solution as defined in the appended claims

Embodiments of the present disclosure have the following advantages:

According to embodiment of the present disclosure, the specific receptor of 2-pentanone is expressed in cultured cells or animals, and its specific binding to 2-pentanone opens ligand-gated cation channels, resulting in an increase of intracellular calcium ion concentration, depolarization of cell membranes, and generation of electrical activity or endocrine activity. The experimental operation is simple and does not require too many instruments.

When being used in treatment, 2-pentanone and the specific receptor of 2-pentanone according to the present disclosure can activate cells rapidly, producing effects with a rapid onset; once the treatment is stopped, the experimental effect can be quickly terminated to allow the cells to return to their original state quickly; the treatment can be performed for a long time, since it does not harm the cells, even in the case of a prolonged treatment; and thus they can be easily applied clinically to treat diseases.

The technique according to the present disclosure can be operated simply, rapidly produce effects which also can be terminated rapidly, and is reusable, so it is promising to be applied clinically.

### BRIEF DESCRIPTION OF THE DRAWINGS

Hereinafter, to more clearly illustrate the technical solutions in the embodiments of the present disclosure or the existing technology, a brief introduction will be made to the drawings necessary to illustrate the embodiments or the existing technology. But, it is obvious that the drawings below are only exemplary and that it is possible for these skilled in the art to derive other implementation drawings from them without any inventive effort.
FIG. 1 is a schematic diagram showing use of 2-pentanone and a 2-pentanone receptor according to an embodiment of the present disclosure;
FIG. 2 is a schematic diagram of an Or35a-P2A-Or83b-P2A-GV320 expression plasmid vector constructed according to an embodiment of the present disclosure;
FIG. 3 is a graph showing the results of detecting 2-pentanone by HPLC-MS according to an embodiment of the present disclosure;
FIG. 4A is a graph showing the results of calcium imaging experiment after the expression of the specific receptor of 2-pentanone in cells according to an embodiment of the present disclosure;
FIG. 4B is a graph showing the results of patch clamp experiment after the expression of the specific receptor of 2-pentanone in cells according to an embodiment of the present disclosure; and
FIG. 5 is a graph showing the results of PCR and Western blot identification of tissues from the F0 generation mouse in an embodiment of the present disclosure.

### DETAILED DESCRIPTION

The embodiments of the present disclosure will be illustrated below using specific examples. Those familiar with this technology can easily understand other advantages and effects of the present disclosure from the contents disclosed in this specification. It is apparent that the described embodiments are only some rather than all of the embodiments of the present disclosure.

### Example 1. Construction of expression vectors of the 2-pentanone specific receptor Or35a and specific receptor Or83b

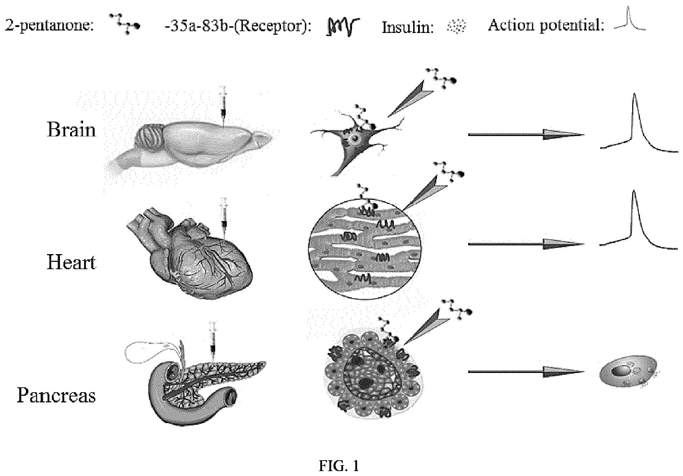

As shown in FIG. 1, the specific receptor of 2-pentanone according to the present disclosure can be expressed in a target area of an animal via a viral vector or by the transgenic technology. When reaching cells in the receptor-expressing area through inhalation and binding to the receptor, 2-pentanone opens ligand-gated cation channels, which results in an increase in intracellular calcium ion concentration, depolarization of cell membranes, generation of electrical activity or endocrine activity, and the other activities.

The expression vectors of the 2-pentanone specific receptor Or35a and specific receptor Or83b in this example were constructed by the following steps:

### I. Construction of Or35a-P2A-Or83b-P2A co-expression gene

1. The extracted cDNA of Drosophila olfactory bulb was used as the template. The nucleotide sequence of the gene of the specific receptor Or35a is shown in SEQ ID NO: 1, and the nucleotide sequence of the gene of the specific receptor Or83b is shown in SEQ ID NO: 2. The amino acid sequence of the protein encoded by the co-expressed sequence of Or35a-P2A-Or83b-P2A gene is shown in SEQ ID NO: 3, the amino acid sequence of the specific receptor 35a is shown in SEQ ID NO: 4, and the amino acid sequence of the specific receptor Or83b is shown in SEQ ID NO: 5. Primers are respectively designed for PCR of Or35a-P2A and Or83b-P2A. Note that the target sequence may also be obtained by direct manual nucleotide-by-nucleotide synthesis according to the sequence.

The primer sequences for amplifying the gene of the receptor Or35a are:
Or35a-P2A-F:
   (XbaI)GCTCTAGAATGGTTCGTTACGTGCCCC, as shown in SEQ ID NO: 6;
Or35a-P2A-R:
   AGGTCCAGGGTTCTCCTCCACGTCTCCAGCCTGCTTCAGCAGGCTGAAGTTA GTAGCATTGCTCATTTGTCGTCGCTG, as shown in SEQ ID NO: 7;

The primer sequences for amplifying the gene of the receptor Or83b are:
Or83b-P2A-F:
   GCTACTAACTTCAGCCTGCTGAAGCAGGCTGGAGACGTGGAGGAGAACCCT GGACCTATGACAACCTCGATGCAGCC, as shown in SEQ ID NO: 8;
Or83b-P2A-R:
   (AgeI)CCACCGGTAGGTCCAGGGTTCTCCTCCACGTCTCCAGCCTGCTTCAGC AGGCTGAAGTTAGTAGCCTTGAGCTGCACCAGCAC, as shown in SEQ ID NO: 9;

Reaction system of PCR amplification included ddH₂O to 50 µl, 25 µl of 2*Phanta Max Master Mix, 2 µl of cDNA, 2 µl of an upstream primer (10 µM), and 2 µl of a downstream primer (10 µM). PCR amplification conditions: 1. Pre-denaturation at 95°C for 3min; 2. Denaturation at 95°C for 15sec; 3. Annealing at 55-60°C for 15 sec; 4. Extension at 72°C and 1min/kb; 5. Final extension at 72°C for 5min; 6. 4°C +∞ at a total of 30-35 cycles.

2. Purification of PCR products: The above PCR products were purified by using the kit (EasyPure PCR Purification Kit, Cat: EP101-02, Beijing TransGen Biotechnology Co. Ltd), and named as product A and product B, respectively. The product A and product B were used as templates to amplify the gene fragment of the co-expressed gene Or35a-P2A-Or83b-P2A using the Overlay PCR method and the following primer sequences.
Or35a-P2A-Or83b-P2A-F: GCTCTAGAATGGTTCGTTACGTGCCCC(XbaI), as shown in SEQ ID NO: 10
Or35a-P2A-Or83b-P2A-R:
   CCACCGGTAGGTCCAGGGTTCTCCTCCACGTCTCCAGCCTGCTTCAGCAGGC TGAAGTTAGTAGCCTTGAGCTGCACCAGCAC(AgeI), as shown in SEQ ID NO: 11

The reaction system included ddH₂O to 50 µl, 25 µl of 2×Phanta Max Master Mix, 2 µl of each of Product A and Product B, 2 µl of an upstream primer (10 µM), and 2 µl of a downstream primer (10 µM). PCR amplification conditions: 1. Pre-denaturation at 95°C for 3min; 2. Denaturation at 95°C for15sec; 3. Annealing at 67°C for 15 sec; 4. Extension at 72°C for 3min; decrease by 0.5°C per cycle; 5. Denaturation at 95°C for 15sec; 6. Annealing at 57°C for 15 sec; 7. Extension at 72°C for 3min; 8. Final extension at 72°C for 5min; 9.4°C +∞. Reagents: vazyme 2×Phanta Max Master Mix, Cat: P515-03.

3. The Or35a-P2A-Or83b-P2A gene fragment and the GV320 plasmid vector were digested by XbaI and AgeI, ligated by T4 ligase at a certain ratio, and then incubated at 16°C overnight to generate the ligation product of the Or35a-P2A-Or83b-P2A gene fragment and the GV320 plasmid vector.

4. The ligation product of the Or35a-P2A-Or83b-P2A gene fragment and the GV320 plasmid vector were transformed into E. coli Stbl3 competent cells, and the plate was inverted at 37°C overnight.

5. Single colonies were picked, inoculated in LB (Amp+) liquid medium and cultured overnight with shaking at 37°C. Plasmid extraction was then performed on the bacterial solution using a kit.

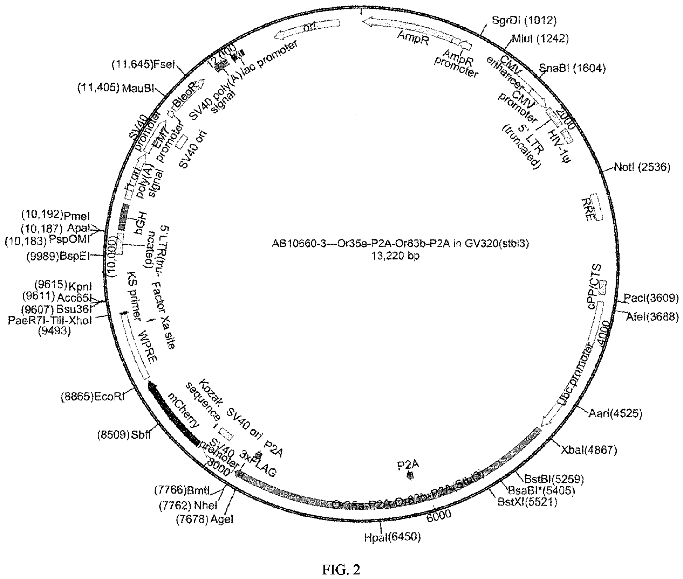

6. The extracted plasmids were preliminarily identified by enzyme digestion and sent for sequencing together with designed primers. The plasmid with correct sequencing was the Or35a-P2A-Or83b-P2A-GV320 expression plasmid vector. The constructed Or35a-P2A-Or83b-P2A-GV320 expression plasmid vector is shown in FIG. 2.

### II. Viral packaging of the expression plasmid vectors

1. HEK 293T cells in good cell condition and endotoxin-free large-scale-extracted recombinant virus core and helper plasmids were prepared. The backbone plasmid Or35a-P2A-Or83b-P2A-GV320 and the shuttle plasmids (pLP1, pLP2 and pLP/VSVG combined) were 16 µg each.
2. Twenty-four hours before transfection, the cells were plated and cultured in a 37°C, 5% CO₂ incubator. After 24h, HEK293T cells were used for transfection when the cell density reached 70%-80%.
3. HEK293T cell culture medium was changed to serum-free medium 2 h before transfection.
4. The recombinant virus core plasmid and helper packaging plasmid were transfected with the corresponding transfection reagents in a certain ratio. First, 32 µg of the viral vector plasmid to be transfected was dissolved in Opti-MEM medium to a total volume of 500 µl, gently mixed and allowed to stand for 5 min. Secondly, the transfection reagent PEI was dissolved in Opti-MEM medium in a ratio of 1:3 with a total volume of 500 µl, gently mixed and allowed to stand for 5 min. Next, the diluted transfection reagent was added dropwise into the plasmid diluent, mixed gently during the addition, and incubated at room temperature for 20 min to allow the DNA and transfection reagent to fully combine to form a stable transfection complex. Then, the cell culture plate was taken out, added with the prepared DNA-PEI complex and placed back to the incubator for further culture. Finally, 3-5h after transfection, 1/2 volume of growth medium containing 30% serum was added for further culture, and after 12h, the transfection medium was removed and replaced with 10ml of fresh complete medium.
5. After 48h of transfection, the cell supernatant enriched with lentiviral particles was collected, concentrated and tested for titer. The lentivirus expressing the specific receptor Or35a and the specific receptor Or83b with titer of ≥ 1×10⁸ TU/mL was obtained.

In this example, expression elements were ectopically expressed in cultured cells or in vivo using plasmid/viral vector or transgenic technology to generate functional Or35a and Or83b co-expressed receptors, i.e., lentivirus expressing the specific receptor Or35a and the specific receptor Or83b. The binding of 2-pentanone to Or35a and Or83b receptors on the cell surface opens ligand-gated cation channels, resulting in an increase of intracellular calcium ion concentration, depolarization of cell membranes, generation of electrical activity or endocrine activity, and other activities.

### Example 2. Construction of transgenic model animals

In this example, the construction process of transgenic model animals is provided as follows:
1. Construction of homologous recombination vectors: A homologous recombination vector was constructed via CRISPR/Cas9 pathway. The exogenous gene Or35a-P2A-Or83b-P2A fragment was inserted between the homologous arms of Rosa26 gene, and the strong CAG promoter with broad expression profile was selected. After a series of enzyme digestion and identification, the vector with 100% homology verified by sequencing was considered as successfully constructed targeting vector.

Construction and identification of SgRNA vector: (1) gRNA primers were synthesized according to Rosa26 site sequence; (2) Single-stranded oligo was phosphorylated and annealed to form a double strand, which was denatured at 98°C and then cooled to room temperature to form a DNA fragment with a sticky end; (3) Plasmid PX459 was cut with Bbs I, recovered after electrophoresis and ligated with annealing primers. (4) The ligation product was transformed into competent cells and plated overnight. (5) Single colonies were picked to screen positive clones by colony PCR; (6) The identified positive clones were verified by sequencing; (7) The correct sequencing results indicate that the CRISPR-SgRNA vector was successfully constructed.

Construction of CRISPR/Cas9 vector: The target Cas9 fragment was amplified by PCR and the recovered PCR product was ligated into Nhe I-digested oriP-EBNA 1 vector. The ligation product was transformed into DH-5α competent cells at 42°C, and cells were cultured overnight on agarose plates containing ampicillin for screening. Single colonies were picked to extract the plasmid DNA, and the insert sequence was verified by sequencing.

Construction of homologous arm vector: About 1-2 kb of sequences targeting both ends of Rosa26 site were amplified respectively by PCR as homologous arms, and IRES-Or35a-P2A-Or83b-P2A fragment was also amplified. Left Arm, right Arm and IRES-GFP fragments were ligated into oriP-EBNA1 vector by seamless cloning. The ligation product was transformed into DH-5a competent cells at 42°C, and cells were cultured overnight on agarose plates containing ampicillin for screening. Single colonies were picked to extract the plasmid DNA, and the insert sequence was verified by sequencing.

2. Detection of site-specific integration vector-associated cells: Cas9 vector, sgRNA vector and IRES-Or35a-P2A-Or83b-P2A homologous recombination vector were introduced into mouse embryonic fibroblasts (MEF) by the electroporation transfection method. After 48h of culture, the genome was extracted. Two pairs of primers were used to detect whether the target gene Or35a-P2A-Or83b-P2A was successfully integrated into the cell genome and inserted into the target site as expected. The PCR product was sequenced for verification.

3. The sgRNA vector, IRES-Or35a-P2A-Or83b-P2A homologous recombination targeting vector and Cas9 vector, which were constructed based on the mechanism of CRISPR/Cas9 system, were injected into the pronuclei of mouse fertilized eggs by pronuclear microinjection.

4. The injected fertilized eggs were transferred to surrogate recipients by embryo transfer technology until the mice were born. The process included that female C57BL/6 mice aged 5-6 weeks were selected and injected intraperitoneally with pregnant mare serum gonadotropin (PMSG) at 5 IU/mouse at 1:00-2:00 pm, and injected intraperitoneally with human chorionic gonadotropin (HCG) at 5 IU/mouse after 46-48 h. Then, they were cohabited and mated with fertile male mice, and the female mice with vaginal plug were selected on the next morning to take fertilized eggs.

The mice were sacrificed. A small segment of uterus with intact oviduct was cut off after stripping and placed in M2 culture medium. The distended ampulla of oviduct was found under a dissecting microscope. By tearing it open with thin pointed forceps, the eggs with cumulus cells flowed out. The fertilized eggs with cumulus cells were then transferred into M2 culture medium containing 1 mg/ml hyaluronidase to lyse the cumulus cells, and the eggs were immediately pipetted into fresh M2 culture medium with a previously pulled pipette to wash 2-3 times and then pipetted into another fresh M2 culture medium. This was done 4-5 times until there were no tissue debris and blood cells in the field of view. Finally, the fertilized eggs were pipetted into the drop (about 50 µl) of M16 culture medium which had been balanced with CO₂, and then the drop was covered with a layer of embryo-grade paraffin oil and placed in the incubator for culture. After the pronucleus of eggs was clearly and fully developed, microinjection could be performed. A capillary glass tube with an outer diameter of 1 mm and an inner diameter of 0.75-0.8 mm was selected and installed on a horizontal micropipette puller to draw an injection needle and egg-holding needle.

A drop of about 50 µl of M2 culture medium was added in the center of 35 cm culture dish, and 5 ml of liquid paraffin oil was added. The culture medium drop was adjusted to be at the center of microscope field. Then 10-20 fertilized eggs with clear nucleus were aspirated and add to the center of the drop. The syringe of the egg-holding needle was adjusted to form negative pressure in the egg-holding needle to aspirate the fertilized eggs with clear edge, full shape and obvious nucleus, and the position of the fertilized eggs were adjusted by changing the pressure of the syringe, so that the male nucleus of the fertilized egg was directly opposite to the injection needle tip with the minimum distance between the two. The positions of the injection needle and egg-holding needle were adjusted, so that the nucleus and injection needle tip had the best clarity. The lever was pushed to carefully inject so that the needle tip entered the nucleus. The injection pressure was increased until the nucleus apparently swollen. The needle was pulled out gently and quickly until it left the cell. Cas9 mRNA was injected in an amount of 100 ng/µl, gRNA in an amount of 20 ng/µl and Or35a-P2A-Or83b-P2 A donor mRNA in an amount of 100 ng/µl. After injection, the fertilized eggs were returned to M16 culture medium and cultured overnight in a cell culture incubator. On the next day, the two-cell embryo was transferred from M16 culture medium to M2 culture medium with embryo transfer needle and washed once or twice. A new transfer tube was used to inject a small amount of paraffin oil and a little M2 culture medium, and then 10-15 injected two-cell embryos were aspirated to prepare for transplantation.

Female ICR mice (over 6 weeks old) in estrus were selected to be cohabited with male ligated mice, and those with vaginal plug were selected on the next day to be surrogate pregnant mice. After anesthetization, the surrogate mice were clipped free of fur on the back with iris tissue scissors and disinfected with 75% alcohol. A longitudinal incision about 1cm long was cut at 1cm beside the intersection of the posterior median line and the horizontal line of the flat costal margin, and the oviduct was pulled out with forceps. The oviduct and ovarian capsule were carefully torn using microscopic forceps. After the opening of oviduct was found, the embryo transfer tube was quickly inserted to inject the embryo for embryo transplantation. Pseudopregnant mice were incubated and allowed to recover. After parturition, the F₀ generation mice were obtained.

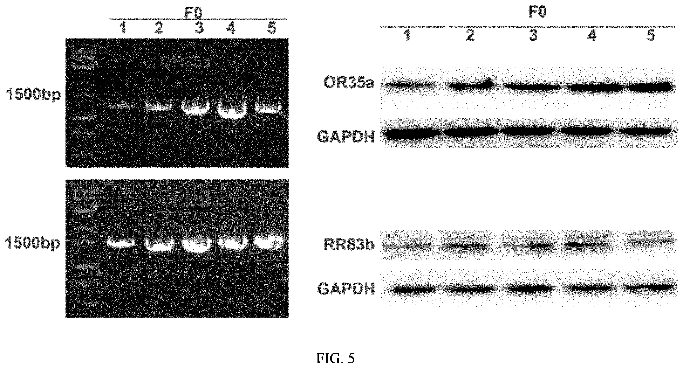

5. The obtained F₀ generation mice were detected preliminarily by PCR and sequencing, and then the target sites may be further sampled for mRNA determination of transcription level and protein determination of translation level. As shown in FIG. 5, tissues from the F0 generation mouse were taken for PCR and Western blot identification, suggesting that the mouse tissues contained the gene of interest.

### Test Example 1. Verification of the arrival of 2-pentanone in cerebrospinal fluid

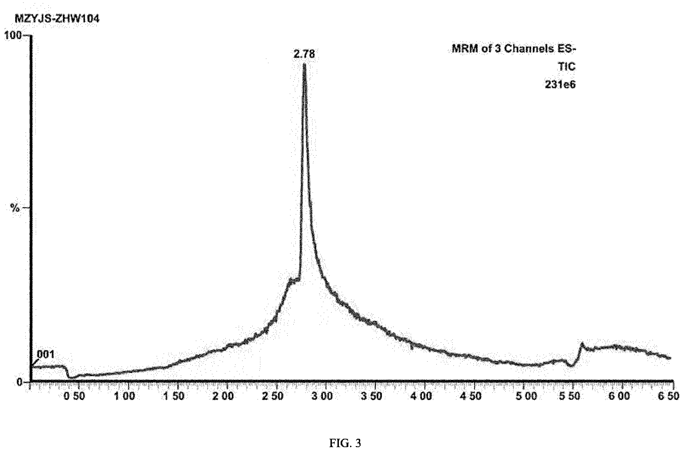

The specific method for detecting the arrival of 2-pentanone in cerebrospinal fluid in this test example included the following: rats were given 2-pentanone for 3 min, then blood and cerebrospinal fluid were drawn, immediately sealed and centrifuged at high speed to pellet red blood cells. The obtained cerebrospinal fluid and blood samples were subjected to HPLC-MS detection. Prior to detection, 100 µL of samples was taken and added with 400 µL of acetonitrile. The protein was separated by vortex and centrifuged at 14000 rpm for 10 min, and 100 µL of supernatant was used for HPLC-MS detection. FIG. 3 shows the results of HPLC-MS detection of 2-pentanone, suggesting that the peak time was 2.72 min. It was confirmed by high performance liquid chromatography-mass spectrometry (HPLC-MS) that 2-pentanone could reach cerebrospinal fluid through blood rapidly (3 min) after inhaled by animals, and the concentration in cerebrospinal fluid decreased rapidly within 3-5min after the inhalation was stopped.

### Test Example 2. Effect of 2-pentanone on cellular calcium content and neuronal firing rate

After the specific receptor of 2-pentanone according to the present disclosure was expressed in cells, the effects of 2-pentanone on cellular calcium content and neuronal firing rate were demonstrated by the calcium imaging experiment and patch clamp experiment.

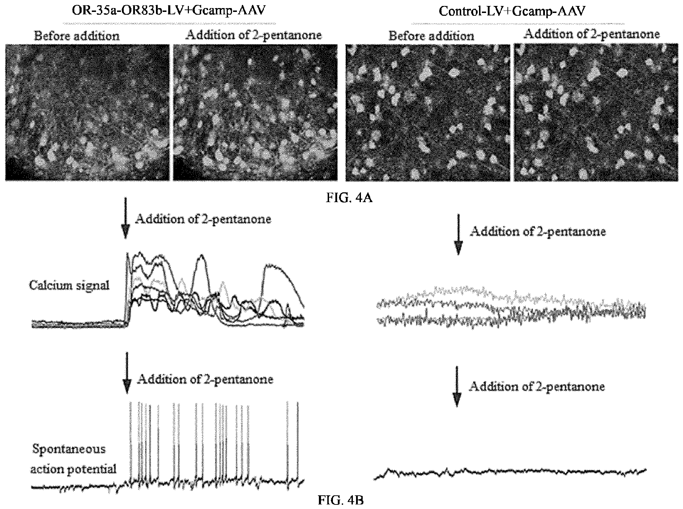

1. Calcium imaging experiment: Adult C57 mice were anesthetized, fixed on a stereotaxic apparatus, and operated to expose the brain surface of the mice (1.5 mm behind the bregma, 1 mm aside, and injection depth of 2 mm). The overexpression virus (1 µl) constructed in Example 1 and the calcium imaging virus (0.5 µl) were simultaneously injected into the mouse brain using a microinjector. After 2 weeks of feeding, the brains of mice were removed. The brain tissues near the injection site were cut into 150 µm tissue slices using a vibrating microtome and placed in artificial cerebrospinal fluid with a mixture of 5% CO₂ and 95% O₂, and calcium imaging experiment was performed using a confocal microscope. As shown in FIG. 4A, after co-expression of the specific receptor of 2-pentanone in cells and tissues, it was confirmed by calcium imaging experiment that the addition of 2-pentanone resulted in a significantly increased intracellular calcium ion concentration.

2. Patch clamp experiment: Adult C57 mice were anesthetized, fixed on a stereotaxic apparatus, and operated to expose the brain surface of the mice (1.5 mm behind the bregma, 1 mm aside, and injection depth of 2 mm). The overexpression virus (1 µl) constructed in Example 1 was injected into the mouse brain using a microinjector. After 2 weeks of feeding, the brains of mice were removed. The brain tissues at and near the injection site were cut into 300 µm tissue slices using a vibrating microtome and placed in artificial cerebrospinal fluid with a mixture of 5% CO₂ and 95% O₂, and after 1 h of incubation, the patch clamp experiment was performed. Using an Axon 700B amplifier, under infrared visualization, neurons with smooth surfaces and clear contours were selected for sealing, and whole-cell recordings were performed. As shown in FIG. 4B, after co-expression of the specific receptor of 2-pentanone in cells and tissues, it was confirmed by patch clamp experiment that the addition of 2-pentanone resulted in a significantly increased neuronal firing rate.

In the present disclosure, the specific receptor of 2-pentanone was expressed in cells of target area and activated by inhaling 2-pentanone. The experimental operation was simple, and 2-pentanone can rapidly reach the target area after being inhaled and can rapidly produce an effect. Inhaled 2-pentanone, most of them, was rapidly excreted through exhalation, and the remaining was cleared by the kidney, enabling rapid metabolism and rapid termination of experimental effects, which was conducive to the observation of experimental phenomena. Besides, since 2-pentanone can rapidly cleared from the body, it can be repeatedly treated several times.

In the present disclosure, by the construction of expression plasmid/virus and the establishment of transgenic animals, the electrical activities of cells was manipulated based on 2-pentanone and specific receptor thereof. In addition, the use of 2-pentanone and specific receptor thereof to manipulate the electrical activity of cells holds promise for applications in the nervous, cardiovascular and skeletal muscle and endocrine systems.

### Sequence Listing

<120> Use of 2-pentanone and Specific Receptor Thereof in Manufacture of Products Regulating Cell Functions
<130> GG19460844A
<160> 5
<170> PatentIn version 3.5
<210> 1
<211> 1230
<212> DNA
<213> Artificial Sequence
<400> 1
   atggttcgtt acgtgccccg gttcgctgat ggtcagaaag taaagttggc ttggcccttg 60
   gcggtttttc ggttaaatca catattctgg ccattggatc cgagcacagg gaaatggggc 120
   cgatatctgg acaaggttct agctgttgcg atgtccttgg tttttatgca acacaacgat 180
   gcagagctga ggtacttgcg cttcgaggca agtaatcgga atttggatgc ctttctcaca 240
   ggaatgccaa cgtatttaat cctcgtggag gctcaattta gaagtcttca cattctactg 300
   cacttcgaga agcttcagaa gtttttagaa atattctacg caaatattta tattgatccc 360
   cgtaaggaac ccgaaatgtt tcgaaaagtg gatggaaaga tgataattaa cagattagtt 420
   tcggccatgt acggtgcagt tatctctctg tatctaatcg cacccgtttt ttccatcatt 480
   aaccaaagca aagattttct atactctatg atctttccgt tcgattcgga tcccttgtac 540
   atatttgtgc cactgctttt gacaaacgta tgggttggca ttgtaataga taccatgatg 600
   ttcggggaga cgaatttgtt gtgtgaacta attgtccacc taaatggtag ttatatgttg 660
   ctcaagaggg acttgcagtt ggccattgaa aagatattag ttgcaaggga ccgtccgcat 720
   atggccaaac agctaaaggt tttaattaca aaaactctcc gaaagaatgt ggctctaaat 780
   cagtttggcc agcagctgga ggctcagtat actgtgcggg tttttattat gtttgcattc 840
   gctgcgggcc ttttatgtgc tctttctttt aaggcttata cgaatcctat ggctaattac 900
   atctatgcga tttggtttgg tgctaagaca gttgagctgc tttctttagg acagattggt 960
   tccgacttgg cctttactac ggattccctc agcacaatgt actaccttac ccattgggag 1020
   caaatcctgc agtactctac aaatcccagc gaaaatctgc gattactaaa gctcattaac 1080
   ttggccattg agatgaacag caagcccttc tatgtgacag ggctaaaata ttttcgcgtt 1140
   agtctgcagg ctggcttaaa aattctgcag gcatccttct cgtacttcac attcctcact 1200
   tcgatgcagc gacgacaaat gagcaattaa 1230
<210> 2
<211> 1461
<212> DNA
<213> Artificial Sequence
<400> 2
   atgacaacct cgatgcagcc gagcaagtac acgggcctgg tcgccgacct gatgcccaac 60
   atccgggcga tgaagtactc cggcctgttc atgcacaact tcacgggcgg cagtgccttc 120
   atgaagaagg tgtactcctc cgtgcacctg gtgttcctcc tcatgcagtt caccttcatc 180
   ctggtcaaca tggccctgaa cgccgaggag gtcaacgagc tgtcgggcaa cacgatcacg 240
   accctcttct tcacccactg catcacgaag tttatctacc tggctgttaa ccagaagaat 300
   ttctacagaa cattgaatat atggaaccag gtgaacacgc atcccttgtt cgccgagtcg 360
   gatgctcgtt accattcgat cgcactggcg aagatgagga agctgttctt tctggtgatg 420
   ctgaccacag tcgcctcggc caccgcctgg accacgatca ccttctttgg cgacagcgta 480
   aaaatggtgg tggaccatga gacgaactcc agcatcccgg tggagatacc ccggctgccg 540
   attaagtcct tctacccgtg gaacgccagc cacggcatgt tctacatgat cagctttgcc 600
   tttcagatct actacgtgct cttctcgatg atccactcca atctatgcga cgtgatgttc 660
   tgctcttggc tgatattcgc ctgcgagcag ctgcagcact tgaagggcat catgaagccg 720
   ctgatggagc tgtccgcctc gctggacacc tacaggccca actcggcggc cctcttcagg 780
   tccctgtcgg ccaactccaa gtcggagcta attcataatg aagaaaagga tcccggcacc 840
   gacatggaca tgtcgggcat ctacagctcg aaagcggatt ggggcgctca gtttcgagca 900
   ccctcgacac tgcagtcctt tggcgggaac gggggcggag gcaacgggtt ggtgaacggc 960
   gctaatccca acgggctgac caaaaagcag gagatgatgg tgcgcagtgc catcaagtac 1020
   tgggtcgagc ggcacaagca cgtggtgcga ctggtggctg ccatcggcga tacttacgga 1080
   gccgccctcc tcctccacat gctgacctcg accatcaagc tgaccctgct ggcataccag 1140
   gccaccaaaa tcaacggagt gaatgtctac gccttcacag tcgtcggata cctaggatac 1200
   gcgctggccc aggtgttcca cttttgcatc tttggcaatc gtctgattga agagagttca 1260
   tccgtcatgg aggccgccta ctcgtgccac tggtacgatg gctccgagga ggccaagacc 1320
   ttcgtccaga tcgtgtgcca gcagtgccag aaggcgatga gcatatcggg agcgaaattc 1380
   ttcaccgtct ccctggattt gtttgcttcg gttctgggtg ccgtcgtcac ctactttatg 1440
   gtgctggtgc agctcaagta a 1461
<210> 3
<211> 933
<212> PRT
<213> Artificial Sequence
<400> 3
   Met Val Arg Tyr Val Pro Arg Phe Ala Asp Gly Gln Lys Val Lys Leu
   1 5 10 15
   Ala Trp Pro Leu Ala Val Phe Arg Leu Asn His Ile Phe Trp Pro Leu
   20 25 30
   Asp Pro Ser Thr Gly Lys Trp Gly Arg Tyr Leu Asp Lys Val Leu Ala
   35 40 45
   Val Ala Met Ser Leu Val Phe Met Gln His Asn Asp Ala Glu Leu Arg
   50 55 60
   Tyr Leu Arg Phe Glu Ala Ser Asn Arg Asn Leu Asp Ala Phe Leu Thr
   65 70 75 80
   Gly Met Pro Thr Tyr Leu Ile Leu Val Glu Ala Gln Phe Arg Ser Leu
   85 90 95
   His Ile Leu Leu His Phe Glu Lys Leu Gln Lys Phe Leu Glu Ile Phe
   100 105 110
   Tyr Ala Asn Ile Tyr Ile Asp Pro Arg Lys Glu Pro Glu Met Phe Arg
   115 120 125
   Lys Val Asp Gly Lys Met Ile Ile Asn Arg Leu Val Ser Ala Met Tyr
   130 135 140
   Gly Ala Val Ile Ser Leu Tyr Leu Ile Ala Pro Val Phe Ser Ile Ile
   145 150 155 160
   Asn Gln Ser Lys Asp Phe Leu Tyr Ser Met Ile Phe Pro Phe Asp Ser
   165 170 175
   Asp Pro Leu Tyr Ile Phe Val Pro Leu Leu Leu Thr Asn Val Trp Val
   180 185 190
   Gly Ile Val Ile Asp Thr Met Met Phe Gly Glu Thr Asn Leu Leu Cys
   195 200 205
   Glu Leu Ile Val His Leu Asn Gly Ser Tyr Met Leu Leu Lys Arg Asp
   210 215 220
   Leu Gln Leu Ala Ile Glu Lys Ile Leu Val Ala Arg Asp Arg Pro His
   225 230 235 240
   Met Ala Lys Gln Leu Lys Val Leu Ile Thr Lys Thr Leu Arg Lys Asn
   245 250 255
   Val Ala Leu Asn Gln Phe Gly Gln Gln Leu Glu Ala Gln Tyr Thr Val
   260 265 270
   Arg Val Phe Ile Met Phe Ala Phe Ala Ala Gly Leu Leu Cys Ala Leu
   275 280 285
   Ser Phe Lys Ala Tyr Thr Asn Pro Met Ala Asn Tyr Ile Tyr Ala Ile
   290 295 300
   Trp Phe Gly Ala Lys Thr Val Glu Leu Leu Ser Leu Gly Gln Ile Gly
   305 310 315 320
   Ser Asp Leu Ala Phe Thr Thr Asp Ser Leu Ser Thr Met Tyr Tyr Leu
   325 330 335
   Thr His Trp Glu Gln Ile Leu Gln Tyr Ser Thr Asn Pro Ser Glu Asn
   340 345 350
   Leu Arg Leu Leu Lys Leu Ile Asn Leu Ala Ile Glu Met Asn Ser Lys
   355 360 365
   Pro Phe Tyr Val Thr Gly Leu Lys Tyr Phe Arg Val Ser Leu Gln Ala
   370 375 380
   Gly Leu Lys Ile Leu Gln Ala Ser Phe Ser Tyr Phe Thr Phe Leu Thr
   385 390 395 400
   Ser Met Gln Arg Arg Gln Met Ser Asn Ala Thr Asn Phe Ser Leu Leu
   405 410 415
   Lys Gln Ala Gly Asp Val Glu Glu Asn Pro Gly Pro Met Thr Thr Ser
   420 425 430
   Met Gln Pro Ser Lys Tyr Thr Gly Leu Val Ala Asp Leu Met Pro Asn
   435 440 445
   Ile Arg Ala Met Lys Tyr Ser Gly Leu Phe Met His Asn Phe Thr Gly
   450 455 460
   Gly Ser Ala Phe Met Lys Lys Val Tyr Ser Ser Val His Leu Val Phe
   465 470 475 480
   Leu Leu Met Gln Phe Thr Phe Ile Leu Val Asn Met Ala Leu Asn Ala
   485 490 495
   Glu Glu Val Asn Glu Leu Ser Gly Asn Thr Ile Thr Thr Leu Phe Phe
   500 505 510
   Thr His Cys Ile Thr Lys Phe Ile Tyr Leu Ala Val Asn Gln Lys Asn
   515 520 525
   Phe Tyr Arg Thr Leu Asn Ile Trp Asn Gln Val Asn Thr His Pro Leu
   530 535 540
   Phe Ala Glu Ser Asp Ala Arg Tyr His Ser Ile Ala Leu Ala Lys Met
   545 550 555 560
   Arg Lys Leu Phe Phe Leu Val Met Leu Thr Thr Val Ala Ser Ala Thr
   565 570 575
   Ala Trp Thr Thr Ile Thr Phe Phe Gly Asp Ser Val Lys Met Val Val
   580 585 590
   Asp His Glu Thr Asn Ser Ser Ile Pro Val Glu Ile Pro Arg Leu Pro
   595 600 605
   Ile Lys Ser Phe Tyr Pro Trp Asn Ala Ser His Gly Met Phe Tyr Met
   610 615 620
   Ile Ser Phe Ala Phe Gln Ile Tyr Tyr Val Leu Phe Ser Met Ile His
   625 630 635 640
   Ser Asn Leu Cys Asp Val Met Phe Cys Ser Trp Leu Ile Phe Ala Cys
   645 650 655
   Glu Gln Leu Gln His Leu Lys Gly Ile Met Lys Pro Leu Met Glu Leu
   660 665 670
   Ser Ala Ser Leu Asp Thr Tyr Arg Pro Asn Ser Ala Ala Leu Phe Arg
   675 680 685
   Ser Leu Ser Ala Asn Ser Lys Ser Glu Leu Ile His Asn Glu Glu Lys
   690 695 700
   Asp Pro Gly Thr Asp Met Asp Met Ser Gly Ile Tyr Ser Ser Lys Ala
   705 710 715 720
   Asp Trp Gly Ala Gln Phe Arg Ala Pro Ser Thr Leu Gln Ser Phe Gly
   725 730 735
   Gly Asn Gly Gly Gly Gly Asn Gly Leu Val Asn Gly Ala Asn Pro Asn
   740 745 750
   Gly Leu Thr Lys Lys Gln Glu Met Met Val Arg Ser Ala Ile Lys Tyr
   755 760 765
   Trp Val Glu Arg His Lys His Val Val Arg Leu Val Ala Ala Ile Gly
   770 775 780
   Asp Thr Tyr Gly Ala Ala Leu Leu Leu His Met Leu Thr Ser Thr Ile
   785 790 795 800
   Lys Leu Thr Leu Leu Ala Tyr Gln Ala Thr Lys Ile Asn Gly Val Asn
   805 810 815
   Val Tyr Ala Phe Thr Val Val Gly Tyr Leu Gly Tyr Ala Leu Ala Gln
   820 825 830
   Val Phe His Phe Cys Ile Phe Gly Asn Arg Leu Ile Glu Glu Ser Ser
   835 840 845
   Ser Val Met Glu Ala Ala Tyr Ser Cys His Trp Tyr Asp Gly Ser Glu
   850 855 860
   Glu Ala Lys Thr Phe Val Gln Ile Val Cys Gln Gln Cys Gln Lys Ala
   865 870 875 880
   Met Ser Ile Ser Gly Ala Lys Phe Phe Thr Val Ser Leu Asp Leu Phe
   885 890 895
   Ala Ser Val Leu Gly Ala Val Val Thr Tyr Phe Met Val Leu Val Gln
   900 905 910
   Leu Lys Ala Thr Asn Phe Ser Leu Leu Lys Gln Ala Gly Asp Val Glu
   915 920 925
   Glu Asn Pro Gly Pro
   930
<210> 4
<211> 409
<212> PRT
<213> Artificial Sequence
<400> 4
   Met Val Arg Tyr Val Pro Arg Phe Ala Asp Gly Gln Lys Val Lys Leu
   1 5 10 15
   Ala Trp Pro Leu Ala Val Phe Arg Leu Asn His Ile Phe Trp Pro Leu
   20 25 30
   Asp Pro Ser Thr Gly Lys Trp Gly Arg Tyr Leu Asp Lys Val Leu Ala
   35 40 45
   Val Ala Met Ser Leu Val Phe Met Gln His Asn Asp Ala Glu Leu Arg
   50 55 60
   Tyr Leu Arg Phe Glu Ala Ser Asn Arg Asn Leu Asp Ala Phe Leu Thr
   65 70 75 80
   Gly Met Pro Thr Tyr Leu Ile Leu Val Glu Ala Gln Phe Arg Ser Leu
   85 90 95
   His Ile Leu Leu His Phe Glu Lys Leu Gln Lys Phe Leu Glu Ile Phe
   100 105 110
   Tyr Ala Asn Ile Tyr Ile Asp Pro Arg Lys Glu Pro Glu Met Phe Arg
   115 120 125
   Lys Val Asp Gly Lys Met Ile Ile Asn Arg Leu Val Ser Ala Met Tyr
   130 135 140
   Gly Ala Val Ile Ser Leu Tyr Leu Ile Ala Pro Val Phe Ser Ile Ile
   145 150 155 160
   Asn Gln Ser Lys Asp Phe Leu Tyr Ser Met Ile Phe Pro Phe Asp Ser
   165 170 175
   Asp Pro Leu Tyr Ile Phe Val Pro Leu Leu Leu Thr Asn Val Trp Val
   180 185 190
   Gly Ile Val Ile Asp Thr Met Met Phe Gly Glu Thr Asn Leu Leu Cys
   195 200 205
   Glu Leu Ile Val His Leu Asn Gly Ser Tyr Met Leu Leu Lys Arg Asp
   210 215 220
   Leu Gln Leu Ala Ile Glu Lys Ile Leu Val Ala Arg Asp Arg Pro His
   225 230 235 240
   Met Ala Lys Gln Leu Lys Val Leu Ile Thr Lys Thr Leu Arg Lys Asn
   245 250 255
   Val Ala Leu Asn Gln Phe Gly Gln Gln Leu Glu Ala Gln Tyr Thr Val
   260 265 270
   Arg Val Phe Ile Met Phe Ala Phe Ala Ala Gly Leu Leu Cys Ala Leu
   275 280 285
   Ser Phe Lys Ala Tyr Thr Asn Pro Met Ala Asn Tyr Ile Tyr Ala Ile
   290 295 300
   Trp Phe Gly Ala Lys Thr Val Glu Leu Leu Ser Leu Gly Gln Ile Gly
   305 310 315 320
   Ser Asp Leu Ala Phe Thr Thr Asp Ser Leu Ser Thr Met Tyr Tyr Leu
   325 330 335
   Thr His Trp Glu Gln Ile Leu Gln Tyr Ser Thr Asn Pro Ser Glu Asn
   340 345 350
   Leu Arg Leu Leu Lys Leu Ile Asn Leu Ala Ile Glu Met Asn Ser Lys
   355 360 365
   Pro Phe Tyr Val Thr Gly Leu Lys Tyr Phe Arg Val Ser Leu Gln Ala
   370 375 380
   Gly Leu Lys Ile Leu Gln Ala Ser Phe Ser Tyr Phe Thr Phe Leu Thr
   385 390 395 400
   Ser Met Gln Arg Arg Gln Met Ser Asn
   405
<210> 5
<211> 486
<212> PRT
<213> Artificial Sequence
<400> 5
   Met Thr Thr Ser Met Gln Pro Ser Lys Tyr Thr Gly Leu Val Ala Asp
   1 5 10 15
   Leu Met Pro Asn Ile Arg Ala Met Lys Tyr Ser Gly Leu Phe Met His
   20 25 30
   Asn Phe Thr Gly Gly Ser Ala Phe Met Lys Lys Val Tyr Ser Ser Val
   35 40 45
   His Leu Val Phe Leu Leu Met Gln Phe Thr Phe Ile Leu Val Asn Met
   50 55 60
   Ala Leu Asn Ala Glu Glu Val Asn Glu Leu Ser Gly Asn Thr Ile Thr
   65 70 75 80
   Thr Leu Phe Phe Thr His Cys Ile Thr Lys Phe Ile Tyr Leu Ala Val
   85 90 95
   Asn Gln Lys Asn Phe Tyr Arg Thr Leu Asn Ile Trp Asn Gln Val Asn
   100 105 110
   Thr His Pro Leu Phe Ala Glu Ser Asp Ala Arg Tyr His Ser Ile Ala
   115 120 125
   Leu Ala Lys Met Arg Lys Leu Phe Phe Leu Val Met Leu Thr Thr Val
   130 135 140
   Ala Ser Ala Thr Ala Trp Thr Thr Ile Thr Phe Phe Gly Asp Ser Val
   145 150 155 160
   Lys Met Val Val Asp His Glu Thr Asn Ser Ser Ile Pro Val Glu Ile
   165 170 175
   Pro Arg Leu Pro Ile Lys Ser Phe Tyr Pro Trp Asn Ala Ser His Gly
   180 185 190
   Met Phe Tyr Met Ile Ser Phe Ala Phe Gln Ile Tyr Tyr Val Leu Phe
   195 200 205
   Ser Met Ile His Ser Asn Leu Cys Asp Val Met Phe Cys Ser Trp Leu
   210 215 220
   Ile Phe Ala Cys Glu Gln Leu Gln His Leu Lys Gly Ile Met Lys Pro
   225 230 235 240
   Leu Met Glu Leu Ser Ala Ser Leu Asp Thr Tyr Arg Pro Asn Ser Ala
   245 250 255
   Ala Leu Phe Arg Ser Leu Ser Ala Asn Ser Lys Ser Glu Leu Ile His
   260 265 270
   Asn Glu Glu Lys Asp Pro Gly Thr Asp Met Asp Met Ser Gly Ile Tyr
   275 280 285
   Ser Ser Lys Ala Asp Trp Gly Ala Gln Phe Arg Ala Pro Ser Thr Leu
   290 295 300
   Gln Ser Phe Gly Gly Asn Gly Gly Gly Gly Asn Gly Leu Val Asn Gly
   305 310 315 320
   Ala Asn Pro Asn Gly Leu Thr Lys Lys Gln Glu Met Met Val Arg Ser
   325 330 335
   Ala Ile Lys Tyr Trp Val Glu Arg His Lys His Val Val Arg Leu Val
   340 345 350
   Ala Ala Ile Gly Asp Thr Tyr Gly Ala Ala Leu Leu Leu His Met Leu
   355 360 365
   Thr Ser Thr Ile Lys Leu Thr Leu Leu Ala Tyr Gln Ala Thr Lys Ile
   370 375 380
   Asn Gly Val Asn Val Tyr Ala Phe Thr Val Val Gly Tyr Leu Gly Tyr
   385 390 395 400
   Ala Leu Ala Gln Val Phe His Phe Cys Ile Phe Gly Asn Arg Leu Ile
   405 410 415
   Glu Glu Ser Ser Ser Val Met Glu Ala Ala Tyr Ser Cys His Trp Tyr
   420 425 430
   Asp Gly Ser Glu Glu Ala Lys Thr Phe Val Gln Ile Val Cys Gln Gln
   435 440 445
   Cys Gln Lys Ala Met Ser Ile Ser Gly Ala Lys Phe Phe Thr Val Ser
   450 455 460
   Leu Asp Leu Phe Ala Ser Val Leu Gly Ala Val Val Thr Tyr Phe Met
   465 470 475 480
   Val Leu Val Gln Leu Lys
   485

## Claims

1. Combination of 2-pentanone and a recombinant vector containing a nucleic acid molecule encoding a specific receptor of the 2-pentanone for use in therapy, wherein said combination promotes an increase in intracellular calcium ion concentration, or an increase in a neuronal firing rate, wherein the specific receptor of the 2-pentanone consists of Or35a having an amino acid sequence set forth in SEQ ID NO: 4 and Or83b having an amino acid sequence set forth in SEQ ID NO: 5, and the recombinant vector is an Or35a-P2A-Or83b-P2A-GV320 expression plasmid vector.

2. The combination according to claim 1 for use in the treatment diseases selected from mental diseases, neurological disorders, myocardial dysfunction, motor dysfunction and endocrine disorders.

3. The combination according to claim 1 or 2, wherein the nucleic acid molecule encoding the specific receptor Or35a has a nucleotide sequence set forth in SEQ ID NO: 1, and the nucleic acid molecule encoding the specific receptor Or83b has a nucleotide sequence set forth in SEQ ID NO: 2.

4. Non-therapeutic use of a combination of 2-pentanone and a recombinant vector containing a nucleic acid molecule encoding a specific receptor of the 2-pentanone for promoting an increase in intracellular calcium ion concentration, or an increase in a neuronal firing rate, wherein the specific receptor of the 2-pentanone consists of Or35a having an amino acid sequence set forth in SEQ ID NO: 4 and Or83b having an amino acid sequence set forth in SEQ ID NO: 5, and the recombinant vector is an Or35a-P2A-Or83b-P2A-GV320 expression plasmid vector.

## Patentansprüche

1. Kombination von 2-Pentanon und einem rekombinanten Vektor, die ein Nukleinsäuremolekül enthält, das einen spezifischen Rezeptor des 2-Pentanons kodiert, und die Anwendung in der Therapie findet, wobei die Kombination eine Erhöhung intrazellulärer Kalziumionenkonzentration oder eine Erhöhung neuronaler Feuerungsrate fördert, wobei der spezifische Rezeptor des 2-Pentanons aus Or35a mit einer Aminosäuresequenz gemäß SEQ ID NO: 4 und Or83b mit einer Aminosäuresequenz gemäß SEQ ID NO: 5 besteht, und der rekombinante Vektor ein Or35a-P2A-Or83b-P2A-GV320-Expressionsplasmidvektor ist.

2. Kombination nach Anspruch 1 zur Verwendung bei der Behandlung von Krankheiten, ausgewählt aus psychischen Krankheiten, neurologischen Störungen, myokardialer Dysfunktion, motorischer Dysfunktion, und Hormonstörungen.

3. Kombination nach Anspruch 1 oder 2, wobei das Nukleinsäuremolekül, das den spezifischen Rezeptor Or35a kodiert, eine Nukleotidsequenz gemäß SEQ ID NO: 1 aufweist und das Nukleinsäuremolekül, das den spezifischen Rezeptor Or83b kodiert, eine Nukleotidsequenz gemäß SEQ ID NO: 2 aufweist.

4. Nichttherapeutische Verwendung einer Kombination von 2-Pentanon und einem rekombinanten Vektor, wobei die Kombination ein Nukleinsäuremolekül enthält, das einen spezifischen Rezeptor des 2-Pentanons kodiert, und wobei die Kombination zur Förderung einer Erhöhung intrazellulärer Kalziumionenkonzentration oder einer Erhöhung neuronaler Feuerungsrate dient, wobei der spezifische Rezeptor des 2-Pentanons aus Or35a mit einer Aminosäuresequenz gemäß SEQ ID NO: 4 und Or83b mit einer Aminosäuresequenz gemäß SEQ ID NO: 5 besteht, und der rekombinante Vektor ein Or35a-P2A-Or83b-P2A-GV320-Expressionsplasmidvektor ist.

## Revendications

1. Combinaison de pentan-2-one et d'un vecteur recombinant contenant une molécule d'acide nucléique codant pour un récepteur spécifique de la pentan-2-one, destinée à être utilisée en thérapie, ladite combinaison favorisant une augmentation de la concentration intracellulaire en ions calcium ou une augmentation d'un taux de décharge neuronale, le récepteur spécifique de la pentan-2-one étant constitué d'Or35a ayant une séquence d'acides aminés définie dans SEQ ID NO: 4 et d'Or83b ayant une séquence d'acides aminés définie dans SEQ ID NO: 5, et le vecteur recombinant étant un vecteur plasmide d'expression Or35a-P2A-Or83b-P2A-GV320.

2. Combinaison selon la revendication 1, destinée au traitement de maladies choisies parmi les maladies mentales, les troubles neurologiques, le dysfonctionnement myocardique, le dysfonctionnement moteur et les troubles endocriniens.

3. Combinaison selon la revendication 1 ou 2, dans laquelle la molécule d'acide nucléique codant pour le récepteur spécifique Or35a a une séquence nucléotidique définie dans SEQ ID NO: 1, et la molécule d'acide nucléique codant pour le récepteur spécifique Or83b a une séquence nucléotidique définie dans SEQ ID NO: 2.

4. Utilisation non thérapeutique d'une combinaison de pentan-2-one et d'un vecteur recombinant contenant une molécule d'acide nucléique codant pour un récepteur spécifique de la pentan-2-one, pour favoriser une augmentation de la concentration intracellulaire en ions calcium ou une augmentation d'un taux de décharge neuronale, le récepteur spécifique de la pentan-2-one étant constitué d'Or35a ayant une séquence d'acides aminés définie dans SEQ ID NO: 4 et d'Or83b ayant une séquence d'acides aminés définie dans SEQ ID NO: 5, et le vecteur recombinant étant un vecteur plasmide d'expression Or35a-P2A-Or83b-P2A-GV320.
